# FASCICULE DE BREVET EUROPEEN

(11) **EP 1 269 979 B1**
(45) Date de publication et mention de la délivrance du brevet: **16.11.2005**
(21) Numéro de dépôt: 02291460.0
(22) Date de dépôt: 12.06.2002
(51) Int. Cl.: A61K 7/42, C07C 255/44, C07C 317/50, C07F 7/08

(54) **Dérivés amides, sulfonamides ou carbamates aromatiques d'acrylonitrile et compositions cosmétiques photoprotectrices les contenant**
Aromatische Amid-, Sulfonamid- oder Karbamatderivate des Acrylsäurenitrils und diese enthaltende kosmetische Lichtschutzzusammensetzungen
Aromatic amide, sulfonamide or carbamate acrylonitrile derivatives and photoprotective cosmetic compositions containing them

(30) Priorité: 20.06.2001 FR 0108092
(43) Date de publication de la demande: 02.01.2003
(73) Titulaire: L'OREAL, 75008 Paris (FR)
(72) Inventeur: Richard, Hervé, 93420 Villepinte (FR); Luppi, Bernadette, 77290 Mitry-Mory (FR)
(74) Mandataire: Casalonga, Axel

(56) Documents cités:
- EP-A- 0 413 648
- EP-A- 0 911 020
- EP-A- 1 005 855
- GB-A- 1 115 596
- DATABASE CA [en ligne] CHEMICAL ABSTRACTS SERVICE, COLUMBUS, OHIO, US; retrieved from STN Database accession no. 1994:163337 XP002198797 & T. HIDAKA ET AL: NIPPON KAGAKU KAISHI, no. 8, 1993, pages 967-972,
- DATABASE CA [en ligne] CHEMICAL ABSTRACTS SERVICE, COLUMBUS, OHIO, US; retrieved from STN Database accession no. 1993:538910 XP002198798 -& CHEMICAL ABSTRACTS, vol. 119, no. 13, 27 septembre 1993 (1993-09-27) Columbus, Ohio, US; abstract no. 138910, XP002198794 & JP 05 058980 A (SEKISUI CHEMICAL CO LTD)
- DATABASE CA [en ligne] CHEMICAL ABSTRACTS SERVICE, COLUMBUS, OHIO, US; retrieved from STN Database accession no. 1992:116719 XP002198799 -& CHEMICAL ABSTRACTS, vol. 116, no. 12, 23 mars 1992 (1992-03-23) Columbus, Ohio, US; abstract no. 116719, XP002198795 & JP 03 206429 A (SEKISUI CHEMICAL CO LTD)
- DATABASE CA [en ligne] CHEMICAL ABSTRACTS SERVICE, COLUMBUS, OHIO, US; retrieved from STN Database accession no. 1993:29821 XP002198800 -& CHEMICAL ABSTRACTS, vol. 118, no. 4, 25 janvier 1993 (1993-01-25) Columbus, Ohio, US; abstract no. 29821, XP002198796 & JP 04 116639 A (KONICA CO)

## Description

La présente invention concerne des compositions cosmétiques à usage topique, en particulier destinées à la photoprotection de la peau et/ou des cheveux, comprenant une quantité efficace d'au moins un dérivé amide, sulfonamide ou carbamate aromatique d'acrylonitrile, de nouveaux dérivés amides, sulfonamides ou carbamates aromatiques d'acrylonitrile, ainsi qu'un procédé de traitement non thérapeutique de la peau utilisant ces compositions.

On sait que les radiations de longueurs d'onde comprises entre 280 nm et 400 nm permettent le brunissement de l'épiderme humain, et que les radiations de longueurs d'onde comprises entre 280 et 320 nm connues sous la dénomination de radiations UV-B, provoquent des érythèmes et des brûlures cutanées qui peuvent nuire au développement du bronzage naturel. Pour ces raisons ainsi que pour des raisons esthétiques, il existe une demande croissante de moyens de contrôle de ce bronzage naturel. Il convient donc de filtrer ce rayonnement UV-B.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible et/ou continuellement exposée au rayonnement solaire. Les rayons UV-A entraînent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photoallergiques. Ainsi, pour des raisons esthétiques et cosmétiques telles que la conservation de l'élasticité naturelle de la peau, de plus en plus de personnes désirent contrôler l'effet des rayons UV-A sur leur peau. Il est donc souhaitable de filtrer aussi le rayonnement UV-A.

Les UV-A se divisent en UV-A-1 dits "UV-A longs" de longueurs d'onde comprises entre 340 et 400 nm et en UV-A-2 dits "UV-A courts" de longueurs d'onde comprises entre 320 et 340 nm. Un bon filtre UV-A doit couvrir autant que possible ces deux gammes de longueurs d'onde, c'est-à-dire être un filtre UV-A large, sans pour autant présenter une coloration propre due à l'absorption de la lumière à des longueurs d'ondes du domaine visible. En effet, un problème important lié à l'utilisation en cosmétique de filtres colorés est le tachage des tissus susceptibles de venir en contact avec ces filtres, tels que les serviettes et vêtements.

De nombreux composés destinés à la protection contre le rayonnement UV-A et/ou UV-B de la peau ont été proposés à ce jour.

La plupart d'entre eux sont des composés aromatiques absorbant le rayonnement UV dans la zone comprise entre 280 nm et 315 nm, ou dans la zone comprise entre 315 nm et 400 nm et au-delà, ou bien dans l'ensemble de ces deux zones. Ils sont le plus souvent formulés dans des compositions antisolaires qui se présentent sous forme d'émulsions huile-dans-eau, les filtres organiques lipophiles ou hydrophiles étant présents sous forme dissoute, dans l'une ou l'autre de ces phases, en des quantités appropriées pour obtenir le facteur de protection solaire (FPS) recherché.

On entend par facteur de protection solaire le rapport du temps d'irradiation nécessaire pour atteindre le seuil érythématogène en présence du filtre testé au temps d'irradiation nécessaire pour atteindre ce même seuil en l'absence de filtre.

Outre leur pouvoir filtrant du rayonnement solaire, les composés photoprotecteurs doivent également présenter de bonnes propriétés cosmétiques, une bonne résistance à l'eau et à la transpiration (rémanence), ainsi qu'une photostabilité satisfaisante.

Parmi tous les composés aromatiques dérivés d'acide 2-cyanoacrylique qui ont été proposés à cet effet, on peut citer les composés décrits dans le brevet FR 1 368 808, les demandes internationales WO 99/36049 et WO 97/15279, et ceux décrits dans les demandes de brevet européen EP-A-0911020, EP-A-0 716 089 et EP-A-0 005 182. Aucun de ces filtres ne remplit cependant simultanément les trois conditions d'être un filtre UV large, c'est-à-dire de filtrer à la fois les UV-A longs et les UV-A courts, d'être non coloré et photostable.

On peut citer également le 4-(tert-butyl)-4'-méthoxydibenzoylméthane, vendu sous le nom commercial Parsol® 1789 par la société Hoffmann Laroche qui est un filtre non coloré, UV-A long ayant un large spectre d'absorption dans l'UV-A, mais qui présente l'inconvénient majeur de se dégrader sous l'effet du rayonnement solaire, c'est-à-dire de ne pas être photostable.

La demanderesse a découvert de manière surprenante qu'une famille particulière de dérivés amides, sulfonamides ou carbamates aromatiques d'acrylonitrile présentaient à la fois
- un spectre large d'absorption UV-A, tout en étant des filtres UV-A longs,
- une absence de coloration,
- une photostabilité satisfaisante, et
- de bonnes propriétés cosmétiques,
et constituaient ainsi d'excellents filtres solaires susceptibles d'être utilisés, en remplacement du Parsol® 1789, dans des compositions cosmétiques, en particulier pour protéger la peau et les cheveux contre les effets néfastes du soleil.

La présente invention a par conséquent pour objet une composition cosmétique à usage topique, en particulier destinée à la photoprotection de la peau et/ou des cheveux, contenant, dans un support cosmétiquement acceptable, au moins un composé choisi parmi les dérivés amides, sulfonamides ou carbamates aromatiques d'acrylonitrile de formule (1) ci-après ou les dimères correspondants de formule (2) ci-après.

Quelques composés de formule (1) ci-après sont connus par exemple de DATABASE CA [Online] CHEMICAL ABSTRACTS SERVICE, COLUMBUS, OHIO, US; XP002198797 extrait de STN Database accession no. 1994:163337, abrégé; RN 153355-28-3, 153355-29-4, T. HIDAKA ET AL: NIPPON KAGAKUKAISHI, no. 8, pages 967-972.

La présente invention a aussi pour objet les composés nouveaux de formule (2) ci-dessous.

La présente invention a enfin pour objet un procédé non-thérapeutique de protection de la peau et/ou des cheveux contre le rayonnement solaire consistant à appliquer sur la peau et/ou les cheveux une quantité efficace de la composition cosmétique définie ci-dessus.

Les dérivés amides, sulfonamides ou carbamates aromatiques d'acrylonitrile utilisés en tant que filtres UV-A à spectre large dans les compositions cosmétiques de la présente invention sont des composés correspondant à la formule générale (1) 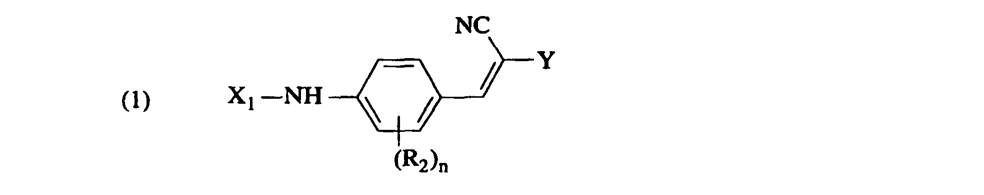 ou des dimères de tels composés, correspondant à la formule (2) : 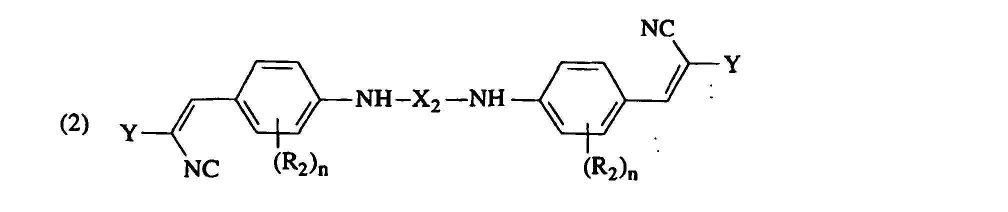 dans lesquelles
X₁ représente un radical R₃-(C=O)- , R₃-SO₂- ou R₃-O-(C=O)-,
X₂ représente un radical divalent de formule -(C=O)-R'₃-(C=O)-, -SO₂-R'₃-SO₂- ou -(C=O)-O-R'₃-O-(C=O)-,
Y représente un radical -(C=O)-R₄ ou -SO₂R₅,
R₂ représente un groupe alkyle en C₁₋₈, linéaire ou ramifié,
n vaut 0, 1 ou 2,
R₃ représente un radical alkyle en C₁₋₃₀ ou alcényle en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,
R'₃ représente une simple liaison ou un radical divalent alkylène en C₁₋₃₀ ou alcénylène en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,.
R₄ représente un radical -OR₆ ou -NHR₆,
R₅ représente un radical alkyle en C₁₋₃₀, linéaire ou ramifié, ou un noyau phényle non substitué ou substitué par des radicaux alkyle ou alcoxy en C₁₋₄,
R₆ représente un radical alkyle en C₁₋₃₀ ou alcényle en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium.

Bien que dans la formule (1) ci-dessus, seuls soient représentés les isomères dans lesquelles le substituant cyano est en position *cis* par rapport au substituant para-amino-phényle, cette formule doit être comprise comme englobant également l'isomère *trans* correspondant. Il en va de même pour la formule (2) dans laquelle, pour chacune des deux double liaisons et de façon indépendante, les substituants cyano et para-amino-phényle peuvent être en configuration *cis* ou *trans* l'un par rapport à l'autre.

Les composés de formule (2) ci-dessus sont des composés nouveaux.

Comme indiqué ci-dessus, les composés de formules (1) et (2) présentent un excellent pouvoir filtrant dans la gamme UV-A large, c'est-à-dire dans le domaine de longueurs d'ondes allant de 320 nm à 400 nm, présentent une photostabilité satisfaisante et sont sensiblement incolores.

Parmi les composés de formules (1) et (2), on préfère en particulier ceux dans lesquels
X₁ représente un radical R₃-(C=O)- ou R₃-O-(C=O)-,
X₂ représente un radical -(C=O)-CH₂-CH₂-(C=O)-,
Y représente un radical -(C=O)-R₄,
n vaut 0,
R₃ représente un radical alkyle en C₁₋₁₀, linéaire ou ramifié,
R₄ représente un radical -O-R₆, et
R₆ représente un groupe alkyle en C₁₋₃₀, linéaire ou ramifié, contenant éventuellement un ou plusieurs atomes de silicium.

Les dérivés de formules (1) et (2) peuvent être préparés selon des méthodes connues.

Pour préparer les dérivés de formule (1), on peut faire réagir 1 mole d'un dérivé de formule (3) sur 1 mole d'un chlorure d'acyle, d'un chlorure de sulfonyle ou d'un chloroformiate de formule (4) en présence de solvant et d'un capteur d'acide chlorhydrique selon le schéma réactionnel suivant : 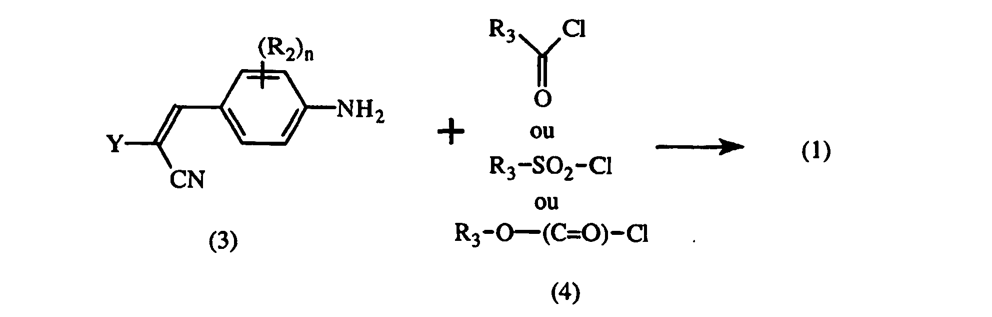 Y, R₂, R₃ et n ayant les significations indiquées précédemment pour la formule (1). Le dérivé de formule (3) peut être préparé par condensation d'un composé de formule (5) ci-dessous avec un dérivé cyano de formule (6) ci-dessous selon le schéma réactionnel : 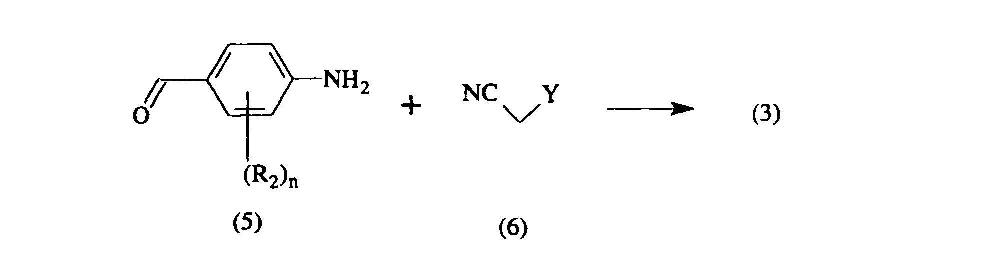 où R₂, Y et n ont les significations indiquées précédemment.
Le composé de formule (1) peut également être préparé par condensation d'un composé de formule (7) ci-dessous avec un dérivé cyano de formule (6) ci-dessous selon le schéma réactionnel suivant : 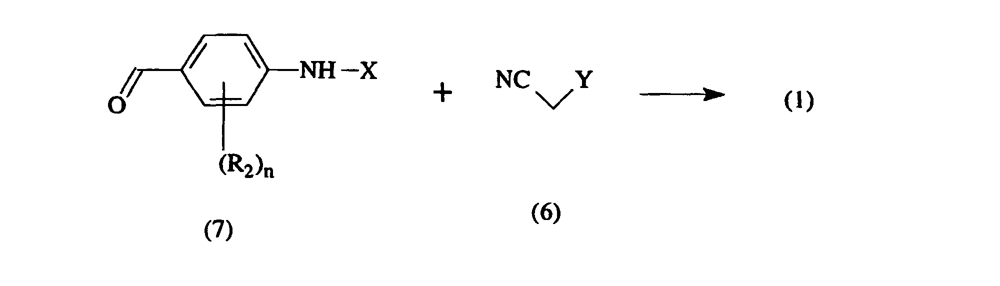 où Y, R₂, X et n ont les significations indiquées précédemment ; le dérivé (7) peut être préparé par condensation d'un dérivé de formule (5) ci-dessous avec un chlorure d'acyle, un chlorure de sulfonyle ou un chloroformiate de formule (4) ci-dessous en présence d'un solvant et d'un capteur d'acide chlorhydrique selon le schéma réactionnel suivant : 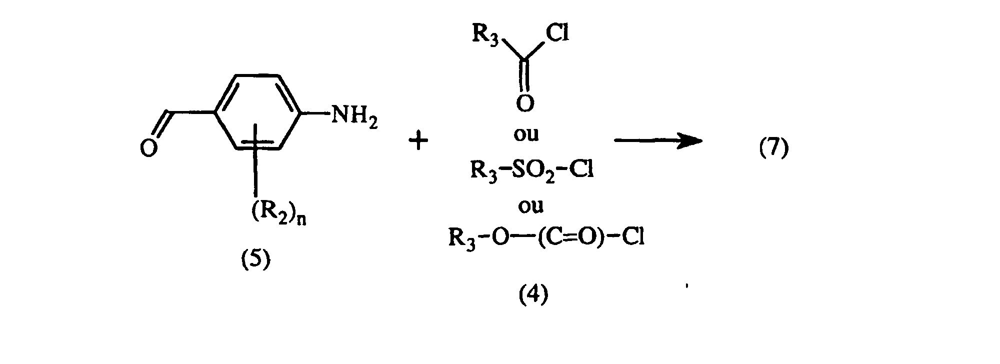 où R₂, R₃ et n ont les significations indiquées précédemment.

Pour préparer les dérivés de formule (2), on peut faire réagir deux moles d'un dérivé de formule (3), préparé de la manière décrite ci-dessus, avec une mole d'un dichlorure d'un acide dicarboxylique ou disulfonique ou d'un dichloroformiate de formule (8) ci-dessous en présence d'un solvant et d'un capteur d'acide chlorhydrique selon le schéma réactionnel suivant : 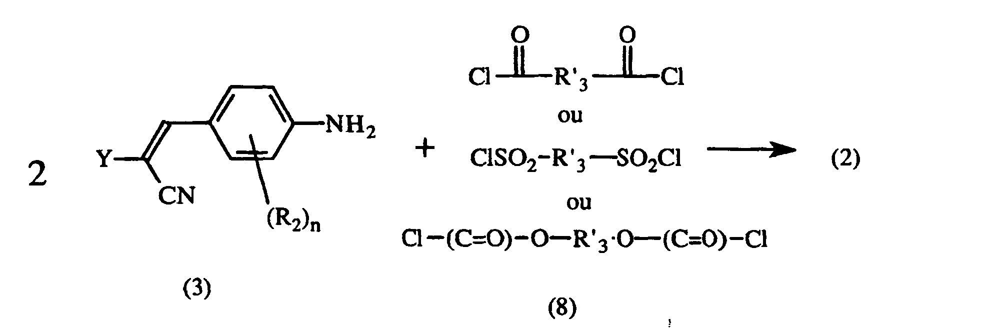 où Y, R₂, R'₃ et n ont les significations indiquées précédemment.

On peut également préparer les composés de formule (2) par condensation d'une mole d'un dérivé de formule (9) ci-dessous avec deux moles d'un dérivé cyano de formule (6) ci-dessous : 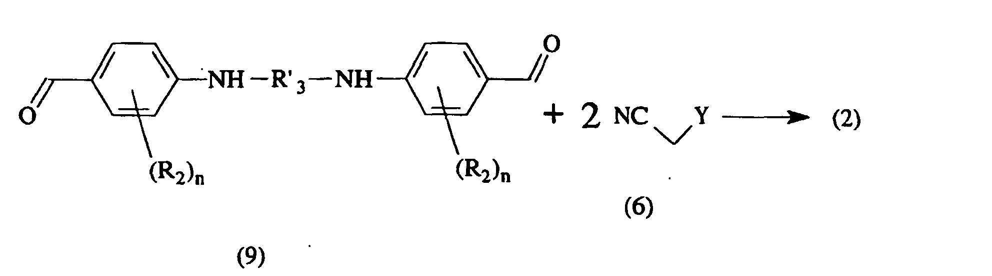 où Y, R₂, R'₃ et n ont les significations indiquées précédemment.

Le dérivé de formule (9) peut être préparé par condensation de deux moles d'un dérivé de formule (5) ci-dessous avec une mole d'un dichlorure d'un acide dicarboxylique ou disulfonique ou d'un dichloroformiate de formule (8) ci-dessous en présence d'un solvant et d'un capteur d'acide chlorhydrique selon le schéma réactionnel suivant : 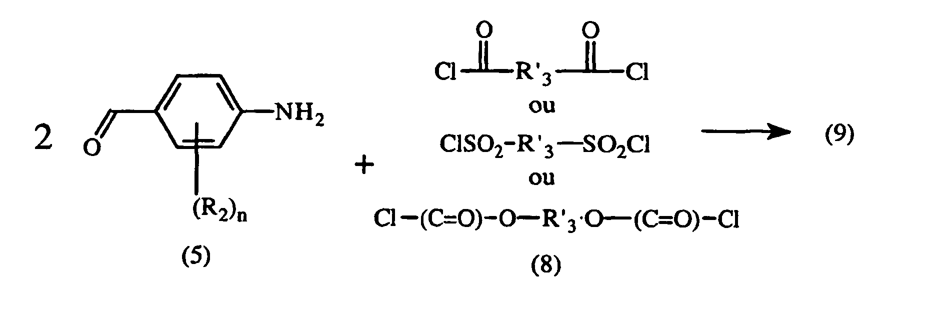 où R₂, R'₃ et n ont les significations indiquées précédemment.

Le ou les composés de formule (1) ou (2) sont de préférence présents dans les compositions cosmétiques à usage topique de la présente invention à raison de 0,1 % et 20 % en poids et en particulier à raison de 0,5 à 10 % en poids, par rapport au poids total de la composition cosmétique.

Les compositions cosmétiques, en particulier anti-solaires, de la présente invention peuvent contenir en outre un ou plusieurs filtres solaires organiques actifs dans le domaine des UV-A et/ou UV-B, différents des filtres UV-A à spectre large de formule (1) ou (2) décrits ci-dessus.

Ces filtres solaires peuvent être notamment choisis parmi les dérivés cinnamiques, les dérivés salicyliques, les dérivés du benzylidènecamphre, les dérivés de triazine tels que ceux décrits dans les brevets ou demandes de brevet US 4 367 390, EP 0 863 145, EP 0 517 104, EP 0 570 838, EP 0 796 851, EP 0 775 698, EP 0 878 469, EP 0 933 376 et EP 0 893 119, les dérivés de la benzophénone, les dérivés de β,β'-diphénylacrylate, les dérivés de phénylbenzimidazole, les dérivés anthraniliques, les dérivés d'imidazolines, les dérivés de méthylène-bis-(hydroxyphénylbenzotriazole) tels que ceux décrits dans les brevets ou demandes de brevet US 5 237 071, US 5 166 355, GB 2 303 549, DE 19726184 et EP 0893 119, les dérivés de l'acide p-aminobenzoïque, les polymères hydrocarbonés filtres et les silicones filtres tels que ceux décrits notamment dans la demande WO 93/04665.

On peut citer à titre d'exemples de tels filtres solaires additionnels actifs dans l'UV-A et/ou l'UV-B, les composés suivants désignés par leur nom INCI, ainsi que leur mélanges :
dérivés d'acide para-aminobenzoïque :
   - PABA
   - Ethyl PABA
   - Ethyl Dihydroxypropyl PABA
   - Ethylhexyl Dimethyl PABA vendu notamment sous le nom commercial ESCALOL® 507 par ISP,
   - Glyceryl PABA,
   - PEG-25 PABA vendu sous le nom commercial UVINUL® P25 par BASF,
dérivés salicyliques :
   - Homosalate vendu sous le nom commercial EUSOLEX® HMS par RONA/EM INDUSTRIES,
   - Ethylhexyl Salicylate vendu sous le nom commercial NEO HELIOPAN® OS par HAARMANN ET REIMER,
   - Dipropyleneglycol Salicylate vendu sous le nom commercial DIPSAL® par SCHER,
   - TEA Salicylate vendu sous le nom commercial NEO HELIOPAN® TS par ET REIMER,
dérivés cinnamiques :
   - Ethylhexyl Methoxycinnamate vendu notamment sous le nom commercial PARSOL® MCX par HOFFMANN LAROCHE,
   - Isopropyl Methoxycinnamate
   - Isoamyl Methoxycinnamate vendu sous le nom commercial NEO HELIOPAN® E 1000 par HAARMANN ET REIMER,
   - Cinoxate,
   - DEA Methoxycinnamate,
   - Diisopropyl Methylcinnamate,
   - Glyceryl Ethylhexanoate Dimethoxycinnamate
dérivés de β,β-diphénylacrylate :
   - Octocrylene vendu notamment sous le nom commercial UVINUL®-539 par BASF,
   - Etocrylene vendu notamment sous le nom commercial UVINUL® N-35 par BASF,
dérivés de benzophénone:
   - Benzophenone-1 vendue sous le nom commercial UVINUL® 400 par BASF,
   - Benzophenone-2 vendue sous le nom commercial UVINUL® D-50 par BASF,
   - Benzophenone-3 ou Oxybenzone vendue sous le nom commercial UVINUL® M-40 par BASF,
   - Benzophenone-4 vendue sous le nom commercial UVINUL® MS-40 par BASF,
   - Benzophenone-5,
   - Benzophenone-6 vendue sous le nom commercial HELISORB® 11 par NORQUAY,
   - Benzophenone-8 vendue sous le nom commercial SPECTRASORB® UV-24 par AMERICAN CYANAMID
   - Benzophenone-9 vendue sous le nom commercial UVINUL® DS-49 par BASF,
   - Benzophénone-12
dérivés du benzylidène camphre :
   - 3-Benzylidene Camphor fabriqué sous le nom MEXORYL® SD par CHIMEX,
   - 4-Methylbenzylidene Camphor vendu notamment sous le nom commercial EUSOLEX® 6300 par MERCK,
   - Benzylidene Camphor Sulfonic Acid fabriqué sous le nom MEXORYL® SL par CHIMEX,
   - Camphor Benzalkonium Methosulfate fabriqué sous le nom MEXORYL® SO par CHIMEX,
   - Terephthalylidene Dicamphor Sulfonic Acid fabriqué sous le nom MEXORYL® SX par CHIMEX,
   - Polyacrylamidomethyl Benzylidene Camphor fabriqué sous le nom MEXORYL® SW par CHIMEX,
dérivés de phénylbenzimidazole :
   - Phenylbenzimidazole Sulfonic Acid vendu notamment sous le nom commercial EUSOLEX® 232 par MERCK
   - Benzimidazilate vendu sous le nom commercial NEO HELIOPAN® AP par HAARMANN ET REIMER
dérivés de triazine :
   - Anisotriazine vendue sous le nom commercial TINOSORB® S par CIBA GEIGY
   - Ethylhexyl triazone vendue notamment sous le nom commercial UVINUL® T150 par BASF,
   - Diéthylhexyl Butamido Triazone vendue sous le nom commercial UVASORB® HEB par SIGMA 3V,
dérivés de phénylbenzotriazole :
   - Drometrizole Trisiloxane vendu sous le nom commercial SILATRIZOLE® par RHODIA CHIMIE,
   - Methylene bis-Benzotriazolyl Tetramethylbutylphenol vendu sous forme solide sous le nom commercial MIXXIM® BB/100 par FAIRMOUNT CHEMICAL ou sous forme micronisée en dispersion aqueuse sous le nom commercial TINOSORB® M par CIBA SPECIALITY CHEMICALS,
dérivés anthraniliques :
   - Menthyl Anthranilate vendu sous le nom commercial NEO HFLIOPAN® MA par HAARMAN ET REIMER,
dérivés d'imidazolines :
   - Ethylhexyl Dimethoxybenzylidene Dioxoimidazoline Propionate
dérivés du benzalmalonate :
   - Polyorganosiloxane à fonctions benzalmalonate vendu sous le nom commercial PARSOL® SLX par HOFFMANN LAROCHE

Les filtres UV organiques plus particulièrement préférés sont choisis parmi les composés suivants :
Ethylhexyl Salicylate,
Ethylhexyl Methoxycinnamate,
Octocrylene,
Phenylbenzimidazole Sulfonic Acid,
Terephthalylidene Dicamphor Sulfonic Acid,
Benzophenone-3,
Benzophenone-4,
Benzophenone-5,
4-Methylbenzylidene Camphor,
Benzimidazilate,
Anisotriazine,
Ethylhexyl Triazone,
Diethylhexyl Butamido Triazone,
Methylene bis-Benzotriazolyl Tetramethylbutylphenol,
Drometrizole Trisiloxane,
et leurs mélanges.

Les compositions cosmétiques selon l'invention peuvent contenir en outre des agents de bronzage et/ou de brunissage artificiel de la peau (agents autobronzants) tels que la dihydroxyacétone (DHA).

Les compositions cosmétiques selon l'invention peuvent contenir en outre un ou plusieurs pigments minéraux et en particulier des nanopigments d'oxydes métalliques, enrobés ou non, comme par exemple les nanopigments d'oxyde de titane sous forme amorphe ou cristallisée (rutile et/ou anatase), d'oxyde de fer, d'oxyde de zinc, d'oxyde de zirconium ou d'oxyde de cérium. Ces nanopigments ont une taille moyenne de particules comprise entre 5 nm et 100 nm, de préférence entre 10 nm et 50 nm et sont tous des agents photoprotecteurs UV connus.

Ces nanopigments peuvent être enrobés par des agents d'enrobage connus comme par exemple l'alumine et/ou le stéarate d'aluminium.

De tels nanopigments enrobés ou non enrobés sont décrits par exemple dans les demandes de brevets EP-A-0 518 772 et EP-A-0 518 773.

Les compositions cosmétiques peuvent contenir en outre des adjuvants habituellement utilisés en cosmétique tels que les corps gras, les solvants organiques, les silicones, les agents tensioactifs, les polymères anioniques, cationiques, non ioniques, amphotères ou leurs mélanges, les agents épaississants, les agents antioxydants, les agents opacifiants, les agents stabilisants, les agents anti-mousse, les parfums, les agents conservateurs, les charges, les agents séquestrants, les agents propulseurs, les agents d'ajustement du pH, les colorants et leurs mélanges.

Elles peuvent contenir en outre un ou plusieurs principes actifs cosmétiques choisis par exemple parmi les agents adoucissants, les hydroxyacides, les vitamines, les agents hydratants, les agents émollients, les agents anti-radicalaires, les antagonistes de substance P, les agents anti-inflammatoires et des mélanges de ces composés.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de palme hydrogénée, l'huile de ricin hydrogénée, l'huile de vaseline, l'huile de paraffine, l'huile de Purcellin, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi, comme la vaseline, la paraffine, la lanoline, la lanoline hydrogénée et la lanoline acétylée.

Parmi les solvants organiques, on peut citer les alcools et polyols inférieurs tels que l'éthanol, l'isopropanol, le propylèneglycol, la glycérine et le sorbitol.

Les épaississants peuvent être choisis notamment parmi les gommes de guar et celluloses, modifiées ou non, telles que la gomme de guar hydroxypropylée, la méthylhydroxyéthylcellulose, l'hydroxypropylméthylcellulose ou encore l'hydroxyéthylcellulose.

Bien entendu l'homme du métier veillera à choisir ce ou ces éventuels composés complémentaires et/ou leurs quantités de manière à ce que les propriétés avantageuses intrinsèques aux composés et compositions selon l'invention ne soient pas altérées par la ou les adjonctions envisagées.

Les compositions cosmétiques à usage topique de l'invention peuvent être préparées selon les techniques bien connues de l'homme du métier, en particulier celles destinées à la préparation d'émulsions de type huile-dans-eau ou eau-dans-huile.

Ces compositions peuvent se présenter en particulier sous forme de lotion, de lotion épaissie, d'émulsion, simple ou complexe (H/E, E/H, H/E/H ou E/H/E), telle qu'une crème ou un lait, sous forme de gel ou de gel-crème, sous forme de poudre ou de bâtonnet solide, et peuvent éventuellement être conditionnées en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins, J. Mol. Biol. 13, 238 (1965), FR 2 315 991 et FR 2 416 008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV ou comme composition antisolaire, elle peut se présenter sous forme de lotion, de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel-crème, de bâtonnet solide, de mousse aérosol ou de spray.

Lorsque la composition cosmétique selon l'invention est utilisée pour la protection des cheveux contre les rayons UV, elle peut se présenter sous forme de shampooing, de lotion, de gel, de composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de composition de permanente ou de défrisage, de coloration ou de décoloration des cheveux ou de laque pour cheveux.

Lorsque la composition est utilisée comme produit de maquillage des cils, des sourcils, de la peau ou des cheveux, tel que crème de traitement de l'épiderme, fond de teint, bâton de rouge à lèvres, fard à paupières, fard à joues, mascara, ligneur encore appelé "eye-liner", gel colorant, elle peut se présenter sous forme solide ou pâteuse, anhydre ou aqueuse, comme une émulsion huile-dans-eau ou eau-dans-huile, une suspension ou un gel.

A titre indicatif, pour les formulations anti-solaires conformes à l'invention, qui présentent un support de type émulsion huile-dans-eau, la phase aqueuse représente généralement de 50 à 95 % en poids, de préférence de 70 à 90 % en poids, par rapport à l'ensemble de la formulation, la phase huileuse de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10 % en poids, par rapport à l'ensemble de la formulation.

Les exemples suivants illustrent l'invention.

### EXEMPLE 1

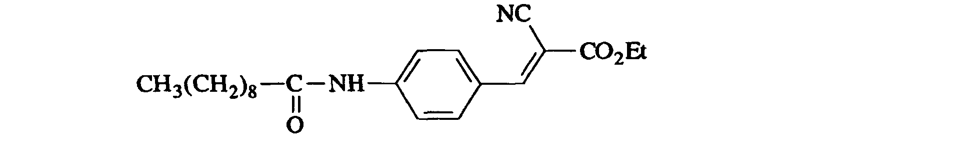

### a) Préparation du 3-(4-aminophényl)-2-cyanoacrylate d'éthyle

On met en suspension 36 g (0,3 mole) de para-aminobenzaldéhyde dans 300 ml d'éthanol. On ajoute à la suspension 33,9 g (0,3 mole) de cyanoacétate d'éthyle et un mélange de 0,375 ml de diéthylamine et de 1,125 ml d'acide acétique, en tant que catalyseur. On chauffe le mélange réactionnel à reflux pendant 2 heures, on laisse refroidir, puis cristalliser. Après séparation des cristaux par filtration et séchage, on obtient 53,9 g (rendement de 83 %) de 3-(4-aminophényl)-2-cyanoacrylate d'éthyle sous forme de cristaux de couleur orange.

### b) Préparation du 2-cyano-3-(4-décanoylaminophényl)acrylate d'éthyle

On dissout 21,6 g (0,1 mole) du composé synthétisé dans l'étape a) dans 300 ml de tétrahydrofurane. On fait couler goutte à goutte à une température de 30 °C, 19 g (0,1 mole) de chlorure d'acide décanoïque. On constate un épaississement progressif du milieu réactionnel. On ajoute ensuite 10,1 g (0,1 mole) de triéthylamine ce qui se traduit par une augmentation de la température à 40 °C. On maintient l'agitation pendant 30 minutes, puis on verse le mélange réactionnel sur 500 ml d'eau. Le précipité obtenu est séparé par filtration, lavé à l'eau, recristallisé dans de l'éthanol et séché. On obtient ainsi 33,5 g (rendement de 84 %) de cristaux de couleur jaune pâle de 2-cyano-3-(4-décanoylaminophényl)acrylate d'éthyle.
Point de fusion : 76 °C
Absorption UV (en solution dans l'éthanol) :
λₘₐₓ = 350 nm, εₘₐₓ = 30 380, E1% = 820

| Analyse élémentaire pour C₂₂ H₃₀ N₂ O₃ | | | | |
|---|---|---|---|---|
| calculé | C 71,32 ; | H 8,16 ; | N 7,56 ; | O 12,96 |
| trouvé | C 71,43 ; | H 8,26 ; | N 7,69 ; | O 13,03 |

### EXEMPLE 2

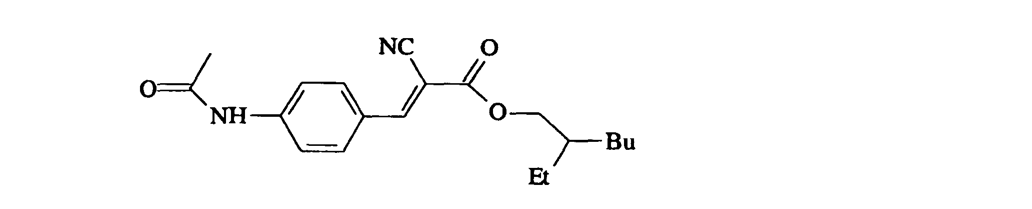

### Préparation du 3-(4-acétylaminophényl)-2-cyanoacrylate de 2-éthylhexyle

On chauffe un mélange de 35 g (0,18 mole) de cyanoacétate de 2-éthylhexyle et de 30 g (0,18 mole) de 4-acétamidobenzaldéhyde dans 300 ml d'isopropanol pendant 3 heures au reflux. On laisse ensuite le mélange réactionnel refroidir et cristalliser. On sépare les cristaux formés par filtration et on recristallise dans un minimum d'isopropanol. On obtient, après filtration et séchage, 33,3 g (rendement de 54 %) de 3-(4-acétylaminophényl)-2-cyanoacrylate de 2-éthylhexyle sous forme d'une poudre jaune pâle.
Point de fusion: 119°C
Absorption UV (en solution dans l'éthanol) :
λₘₐₓ = 351 nm, εₘₐₓ = 30 960, E1% = 904

| Analyse élémentaire pour C₂₀ H₂₆ N₂ O₃ | | | | |
|---|---|---|---|---|
| calculé | C 70,15 ; | H 7,65 ; | N 8,18 ; | O 14,02 |
| trouvé | C 70,08 ; | H 7,65 ; | N 8,16 ; | O 14,03 |

### EXEMPLE 3

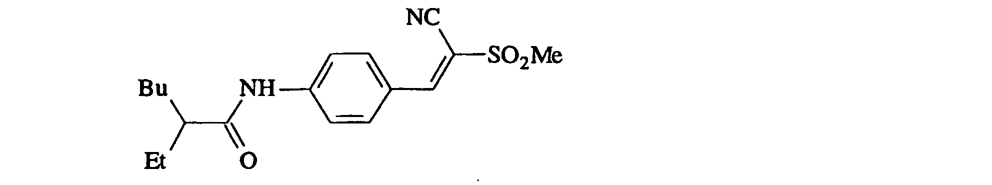

### a) Préparation du 3-(4-aminophényl)-2-méthanesulfonylacrylonitrile

On chauffe un mélange de 11,9 g (0,1 mole) de méthanesulfonylacétonitrile, de 12 g (0,1 mole) de 4-aminobenzaldéhyde, et d'un catalyseur constitué de 0,125 ml de diéthylamine et de 0,375 ml d'acide acétique dans 150 ml d'éthanol, pendant 6 heures au reflux. Après arrêt de la réaction, on élimine les insolubles par filtration à chaud et on concentre le filtrat sous vide, on le laisse refroidir et cristalliser. On obtient, après filtration et séchage des cristaux, 11,6 g (rendement de 52 %) d'une poudre orangée de 3-(4-aminophényl)-2-méthanesulfonylacrylonitrile.

### b) Préparation du 3-[4-(2-éthylhexanoyl)amino-phényl]-2-méthane-sulfonylacrylonitrile

On met en suspension 4 g (0,018 mole) du composé obtenu dans l'étape a) dans 50 ml de tétrahydrofurane. On fait couler goutte à goutte à température ambiante 2,9 g (0,018 mole) de chlorure d'acide 2-éthylhexanoïque ce qui a pour effet une augmentation de la température à 27 °C. On chauffe le mélange à 45 °C, puis on ajoute 1,8 g (0,018 mole) de triéthylamine. On fait réagir pendant 1 heure à 60 °C. On ajoute ensuite 150 ml d'eau et on laisse cristalliser. Le précipité est séparé par filtration, lavé à l'eau et essoré. On le recristallise dans un mélange isopropanol/eau. On obtient ainsi 3,9 g (rendement de 62 %) d'une poudre jaune pâle du composé recherché.
Point de fusion : 178 °C
Absorption UV (en solution dans l'éthanol) :
λₘₐₓ = 348 nm, εₘₐₓ = 33 500, E1% = 960

| Analyse élémentaire pour C₁₈ H₂₄ N₂ O₃ S | | | | | |
|---|---|---|---|---|---|
| calculé | C 62,04 ; | H 6,94 ; | N 8,04 ; | O 13,77 ; | S 9,20 |
| trouvé | C 61,90 ; | H 6,88 ; | N 8,05 ; | O 13,77 ; | S 9,13 |

### EXEMPLE 4

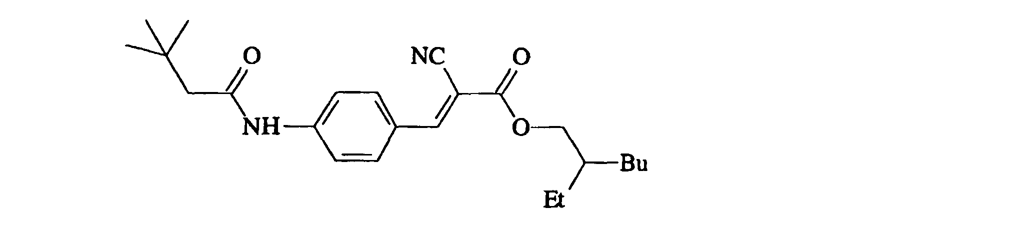

### a) Préparation du 3-(4-aminophényl)-2-cyanoacrylate de 2-éthylhexyle

On met en suspension 36,3 g (0,3 mole) de p-aminobenzaldéhyde dans 450 ml d'isopropanol. On y introduit un mélange de 59,1 g (0,3 mole) de cyanoacétate de 2-éthylhexyle et de catalyseur (0,375 ml de diéthylamine et 1,125 ml d'acide acétique). On chauffe au reflux pendant 5 heures. On ajoute de nouveau la même quantité de catalyseur et on poursuit le chauffage au reflux pendant 4 heures supplémentaires. On élimine les insolubles par filtration à chaud, on concentre le mélange jusqu'à un volume de 300 ml et on laisse cristalliser. On obtient, après filtration et séchage, 59,6 g (rendement de 66 %) d'une poudre orangée utilisée telle quelle pour l'étape suivante.

### b) Préparation du 2-cyano-3-[4-(3.3-diméthyl)butyrylamino)-phényl]-acrylate de 2-éthylhexyle .

On dissout 10 g (0,033 mole) de 3-(4-aminophényl)-2-cyanoacrylate de 2-éthylhexyle dans 50 ml de tétrahydrofurane. On fait couler goutte à goutte à température ambiante 4,48 g (0,033 mole) de chlorure de tert-butylacétyle ce qui a pour effet une augmentation de la température à 29 °C. On chauffe le mélange à environ 45 °C, puis on ajoute 3,3 g (0,033 mole) de triéthylamine. On chauffe à 50 °C pendant 30 minutes. Après retour à température ambiante, on ajoute 150 ml d'eau, puis on extrait avec du dichlorométhane. On lave la phase organique à l'eau, on la sèche et on concentre sous vide. Après recristallisation du solide obtenu dans l'heptane, on obtient 10,6 g (rendement de 81 %) d'une poudre jaune pâle du composé recherché.
Point de fusion : 114 °C
Absorption UV (en solution dans l'éthanol) :
λₘₐₓ = 355 nm, εₘₐₓ = 31900, E1% = 800

| Analyse élémentaire pour C₂₄ H₃₄ N₂ O₃ | | | | |
|---|---|---|---|---|
| calculé | C 72,33 ; | H 8,60 ; | N 7,03 ; | O 12,04 |
| trouvé | C 72,40 ; | H 8,55 ; | N 7,10 ; | O 12,28 |

### EXEMPLE 5

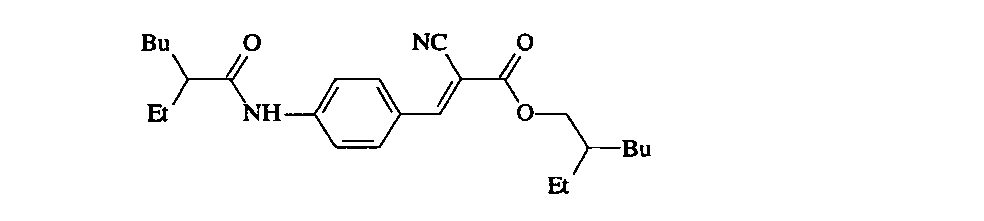

### Préparation de 2-cyano-3 [4-(2-éthylhexanoyl)amino-phényl]-acrylate de 2-éthylhexyle

On dissout 10 g (0,033 mole) de 3-(4-aminophényl)-2-cyanoacrylate de 2-éthylhexyle préparé conformément à l'étape a) de l'exemple 4, dans 50 ml de tétrahydrofurane.

On y ajoute goutte à goutte à température ambiante 5,4 g (0,033 mole) de chlorure d'acide 2-éthylhexanoïque ce qui a pour effet une légère augmentation de la température. On chauffe le mélange réactionnel à 45 °C, puis on ajoute 3,3 g (0,033 mole) de triéthylamine. On poursuit le chauffage du mélange au bain marie pendant 1 heure 30 minutes. Après retour à la température ambiante, on ajoute 150 ml d'eau, on extrait au dichlorométhane et on évapore à sec. On recristallise le solide jaune obtenu dans l'heptane et on obtient 10,8 g (rendement de 77 %) du composé recherché sous forme d'une poudre jaune pâle.
Point de fusion : 68 °C
Absorption UV (en solution dans l'éthanol) :
λₘₐₓ = 353 nm, εₘₐₓ = 34 300, E1% = 804

| Analyse élémentaire pour C₂₆ H₃₈ N₂ O₃ | | | | |
|---|---|---|---|---|
| calculé | C 73,20 ; | H 8,98 ; | N 6,57 ; | O 11,25 |
| trouvé | C 73,43 ; | H 8,86 ; | N 6,68 ; | O 11,45 |

### EXEMPLE 6

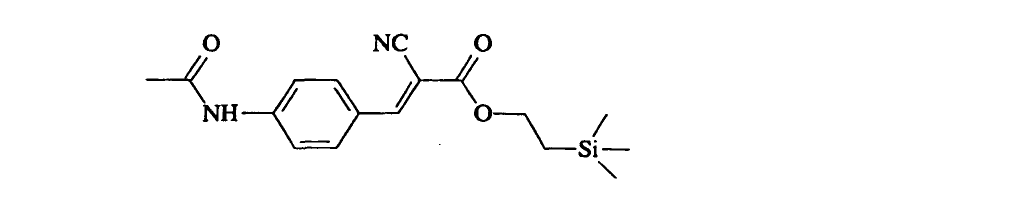

### a) Préparation du 3-(4-acétylaminophényl)-2-cyanoacrylate d'éthyle

On chauffe au reflux un mélange de 33,9 g (0,3 mole) de cyanoacétate d'éthyle et de 59,1 g (0,3 mole) de 4-acétamidobenzlaldéhyde dans 300 ml d'éthanol en présence de 5 ml de pipéridine pendant 3 heures. Le milieu très visqueux est dilué avec 800 ml d'éthanol chaud. Il est filtré à chaud. Le solide jaune obtenu est lavé à l'éthanol chaud. On obtient, après filtration et séchage, 32 g (rendement de 41 %) de 3-(4-acétylaminophényl)-2-cyanoacrylate d'éthyle sous forme d'une poudre jaune pâle.
Point de fusion: 187°C
Absorption UV (en solution dans l'éthanol) :
λₘₐₓ = 350 nm, εₘₐₓ = 31 820 E1% = 1232

| Analyse élémentaire pour C₁₄H₁₄N₂O₃ | | | | |
|---|---|---|---|---|
| calculé | C 65,11 ; | H 5,46 ; | N 10,85 ; | O 18,58 |
| trouvé | C 64,89 ; | H 5,63 ; | N 11,06 ; | O 18,38 |

### b) Préparation du 3-[4-acétylaminophényl]-2-cyanoacrylate de 2-triméthylsilyléthyle

On chauffe au reflux (150 °C) pendant 3 heures 2 g (0,0078 mole) de 3-(4-acétylaminophényl)-2-cyanoacrylate d'éthyle dans 20 ml de 2-triméthylsilyléthanol contenant 0,1 ml de pipéridine.

Le large excès de 2-triméthylsilyléthanol est évaporé à pression normale. Après retour à température ambiante, on ajoute 50 ml d'heptane au résidu. Le solide jaune obtenu après trituration dans l'heptane est filtré, lavé à l'heptane et séché. On obtient 2,1 g (rendement de 78 %) du composé recherché sous forme d'une poudre jaune pâle.
Point de fusion : 136 °C
Absorption UV (en solution dans l'éthanol) :
λₘₐₓ = 349 nm, εₘₐₓ = 27 450, E1% = 830

| Analyse élémentaire pour C₁₇ H₂₂ N₂ O₃ Si | | | | |
|---|---|---|---|---|
| calculé : trouvé : | C 61,79 ; | H 6,71 ; | N 8,48 ; | Si 8,50 |
| | C 61,40 ; | H 6,80 ; | N 8,36 ; | Si 8,15 |

### EXEMPLE 7

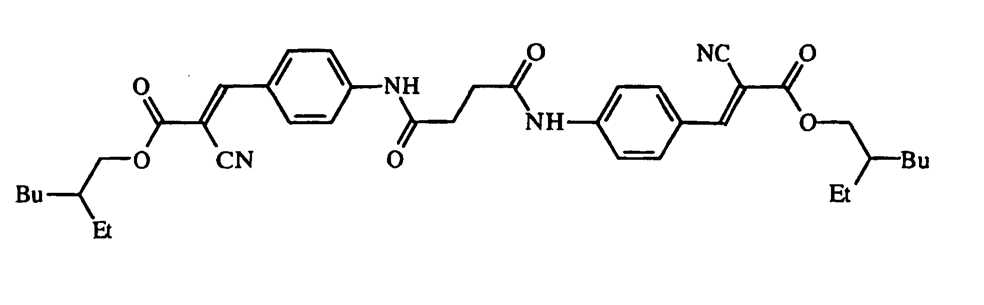

### Préparation du dimère de 2-cyano-3-[4-(acétylamino)phényl]-acrylate de 2-éthylhexyle

On dissout 6 g (0,02 mole) de 3-(4-aminophényl)-2-cyanoacrylate de 2-éthylhexyle synthétisé dans l'étape a) de l'exemple 4 dans 60 ml de tétrahydrofurane. On fait couler goutte à goutte à température ambiante 1,6 g (0,01 mole) de chlorure de succinyle ce qui a pour effet une augmentation de la température à 36 °C. On chauffe le mélange à environ 45 °C, puis on ajoute 2,02 g (0,02 mole) de triéthylamine. On chauffe à 55 °C pendant 3 heures. Le milieu réactionnel devenu visqueux est refroidi à température ambiante, il est filtré. Il est bien lavé à l'eau, bien essoré, puis lavé avec un minimum d'éthanol à 96%. Après séchage du solide obtenu, on obtient 4,6 g (rendement de 67 %) d'une poudre jaune pâle du composé recherché.
Point de fusion : 235 °C
Absorption UV (en solution dans l'éthanol) :
λₘₐₓ = 356 nm, εₘₐₓ=62 620, E1%=917

| Analyse élémentaire pour C₂₄ H₃₄ N₂ O₃, 1/2H₂O | | | | |
|---|---|---|---|---|
| calculé | C 69,44 ; | H 7,43; | N 8,10 ; | O 15,03 |
| trouvé | C 69,71 ; | H 7,39 ; | N 8,36 ; | O 15,01 |

### EXEMPLE 8

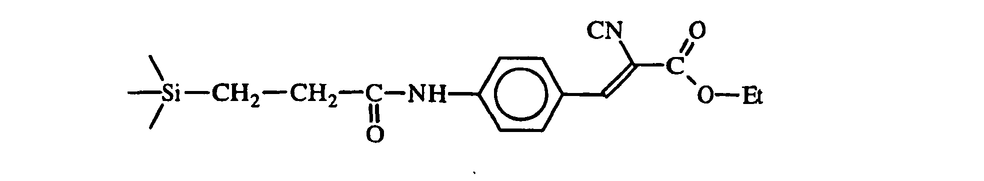

### a) Préparation du chlorure d'acide 3-triméthylsilylpropanoïque

On ajoute goutte à goutte sous agitation à un mélange de 10 g (0,068 mole) d'acide 3-triméthylsilylpropanoïque dans 30 ml de 1,2-dichloroéthane 13,5 g (0,11 mole) de chlorure de thionyle. On chauffe le mélange réactionnel à 40°C pendant 2 heures. L'excès de chlorure de thionyle est éliminé par distillation sous pression réduite. La solution obtenue est utilisée telle quelle pour l'étape suivante.

### b) Préparation de 2-cyano-3[4-(3-triméthylsilylpropanoylamino)-phényl]-acrylate d'éthyle

Dans la solution obtenue précédemment, on introduit par portions à température ambiante une suspension de 13,7 g (0,0633 mole) de 3-(4-aminophényl)-2-cyanoacrylate d'éthyle préparé conformément à l'étape a) de l'exemple 1 dans 80 ml de 1,2-dichloroéthane. On chauffe le mélange réactionnel hétérogène à 40 °C, puis on ajoute goutte à goutte 7,1 g (0,07 mole) de triéthylamine. On poursuit le chauffage entre 45 et 50°C pendant 1 heure. Après retour à la température ambiante, le mélange réactionnel est versé dans 500 ml d'eau. La phase organique est séparée, lavée 2 fois à l'eau, séchée sur du sulfate de sodium et le solvant est évaporé. On recristallise le solide obtenu dans du 1,2-dichloroéthane et on obtient 8,5 g (rendement de 40 %) du composé recherché sous forme d'une poudre jaune pâle.
Point de fusion : 157 °C
Absorption UV (en solution dans l'éthanol) :
λₘₐₓ = 352 nm, εₘₐₓ = 30 730, E1% = 892

| Analyse élémentaire pour C₁₈ H₂₄ N₂ O₃ Si | | | | |
|---|---|---|---|---|
| calculé | C 62,76 ; | H 7,02 ; | N 8,13 ; | Si 8,15 |
| trouvé | C 62,84 ; | H 7,06 ; | N 8,16 ; | Si 7,93 |

### EXEMPLE 9

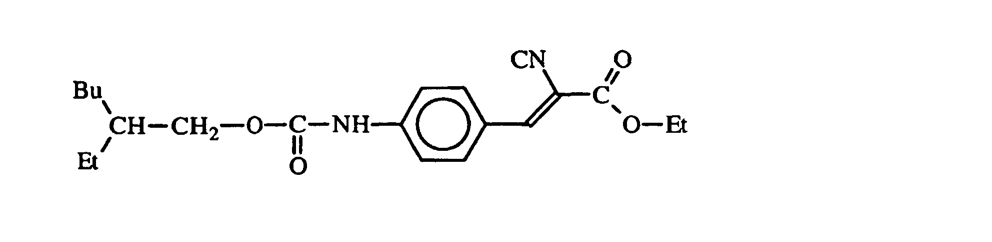

### Préparation de 2-cyano-3 [4-(2-éthylhexyloxycarbonylamino)-phényl]-acrylate d'éthyle

On dissout 4,3 g (0,02 mole) de 3-(4-aminophényl)-2-cyanoacrylate d'éthyle préparé conformément à l'étape a) de l'exemple 1, dans 50 ml de tétrahydrofurane.

On y ajoute goutte à goutte à température ambiante 3,8 g (0,02 mole) de chloroformiate de 2-éthylhexyle, puis on ajoute 2,02 g (0,02 mole) de triéthylamine. On chauffe le mélange à 50 °C au bain marie pendant 2 heures. Après retour à la température ambiante, on ajoute 100 ml d'eau, on extrait au dichlorométhane et on évapore à sec. Le résidu est de nouveau extrait à l'éther éthylique. La pâte obtenue est chromatographiée sur silice (éluant : dichlorométhane). Les fractions contenant le composé recherché sont regroupées. On obtient, après évaporation, une huile qui cristallise. On recristallise le solide obtenu dans l'heptane et on obtient 2,2 g (rendement de 44 %) du composé recherché sous forme d'une poudre jaune pâle.
Point de fusion : 105°C
Absorption UV (en solution dans l'éthanol) :
λₘₐₓ = 355 nm, εₘₐₓ = 31 860, E1% = 856

| Analyse élémentaire pour C₂₁ H₂₈ N₂ O₄ | | | | |
|---|---|---|---|---|
| calculé | C 67,72 ; | H 7,58 ; | N 7,52 ; | O 17,18 |
| trouvé | C 67,78 ; | H 7,45 ; | N 7,81 ; | O 17,05 |

### EXEMPLE 10

### Composition antisolaire (émulsion huile-dans-eau)

- 3-(4-acétylaminophényl)-2-cyanoacrylate de 2-éthylhexyle (composé de l'exemple 2) 4 g
- Mélange d'alcool cétylstéarylique et d'alcool cétylstéarylique oxyéthyléné (33 motifs OE) 80/20 vendu par la société TENSIA sous la dénomination commerciale DEHSCONET® 390 7 g
- Mélange de mono- et de distéarate de glycérol vendu sous la dénomination commerciale CERASYNTH® SD par la société ISP 2 g
- Alcool cétylique 1,5 g
- Polydiméthylsiloxane vendu sous la dénomination DC200Fluid® par la société DOW CORNING 1,5 g
- Benzoate d'alcools en C₁₂₋₁₅ vendu sous la dénomination FINSOLV® TN par la société FINTEX 16 g
- Glycérine 20 %
- Conservateurs q.s. • Eau déminéralisée qsp 100 g

On dissout le filtre solaire et les agents tensioactifs dans la phase grasse chauffée à environ 70 - 80 °C. On ajoute ensuite sous agitation vigoureuse l'eau chauffée à la même température. On maintient l'agitation pendant 10 à 15 minutes, puis on laisse refroidir sous agitation modérée jusqu'à environ 40 °C et on ajoute les conservateurs. On obtient ainsi une crème anti-solaire particulièrement efficace contre les UV-A et qui ne tache pas les substrats avec lesquels elle est mise en contact.

## Revendications

1. Composition cosmétique à usage topique, en particulier destinée à la photoprotection de la peau et/ou des cheveux, contenant, dans un support cosmétiquement acceptable, au moins un composé de formule (1) ou (2) 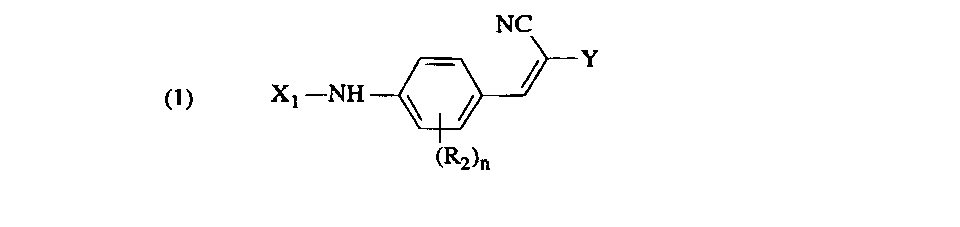 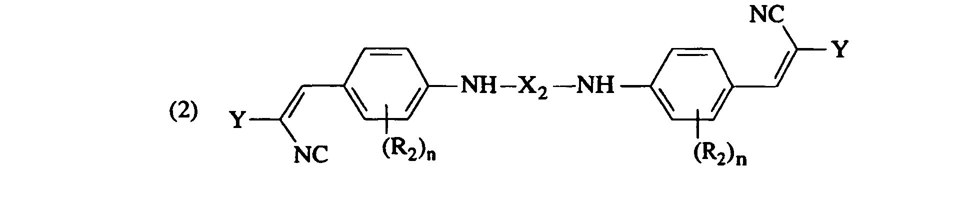 dans lesquelles
X₁ représente un radical R₃-(C=O)- , R₃-SO₂- ou R₃-O-(C=O)-,
X₂ représente un radical divalent de formule -(C=O)-R'₃-(C=O)-, -SO₂-R'₃-SO₂- ou -(C=O)-O-R'₃-O-(C=O)-,
Y représente un radical -(C=O)-R₄ ou -SO₂R₅,
R₂ représente un groupe alkyle en C₁₋₈, linéaire ou ramifié,
n vaut 0, 1 ou 2,
R₃ représente un radical alkyle en C₁₋₃₀ ou alcényle en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,
R'₃ représente une simple liaison ou un radical divalent alkylène en C₁₋₃₀ ou alcénylène en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,.
R₄ représente un radical -OR₆ ou -NHR₆,
R₅ représente un radical alkyle en C₁₋₃₀, linéaire ou ramifié, ou un noyau phényle non substitué ou substitué par des radicaux alkyle ou alcoxy en C₁₋₄,
R₆ représente un radical alkyle en C₁₋₃₀ ou alcényle en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium.

2. Composition cosmétique selon la revendication 1, **caractérisée par le fait que**
X₁ représente un radical R₃-(C=O)- ou R₃-O-(C=O)-,
X₂ représente un radical -(C-O)-CH₂-CH₂-(C=O)-,
Y représente un radical -(C=O)-R₄,
n vaut 0,
R₃ représente un radical alkyle en C₁₋₁₀, linéaire ou ramifié,
R₄ représente un radical -O-R₆, et
R₆ représente un radical alkyle en C₁₋₃₀, linéaire ou ramifié, contenant éventuellement un ou plusieurs atomes de silicium.

3. Composition cosmétique selon la revendication 1 ou 2, **caractérisée par le fait que** le ou les composés de formule (1) ou (2) sont présents à raison de 0,1 % à 20 % en poids et de préférence à raison de 0,5 à 10 % en poids, par rapport au poids total de la composition cosmétique.

4. Composition cosmétique selon l'une quelconque des revendications précédentes, **caractérisée par le fait qu'**elle contient en outre un ou plusieurs filtres solaires organiques actifs dans le domaine des UV-A et/ou UV-B, différents de ceux de formule (1) ou (2).

5. Composition cosmétique selon la revendication 4, **caractérisée par le fait que** les filtres solaires organiques actifs dans le domaine des UV-A et/ou UV-B, différents de ceux de formule (1) et (2), sont choisis parmi les dérivés salicyliques, les dérivés du benzylidènecamphre, les dérivés de triazine, les dérivés de la benzophénone, les dérivés cinnamiques, les dérivés de β,β'-diphénylacrylate, les dérivés de phénylbenzimidazole, les dérivés anthraniliques, les dérivés d'imidazolines, les dérivés de méthylène-bis-(hydroxyphénylbenzotriazole), les dérivés de l'acide p-aminobenzoïque, les polymères hydrocarbonés filtres et les silicones filtres.

6. Composition cosmétique selon la revendication 5, **caractérisée par le fait que** les filtres solaires organiques actifs dans le domaine des UV-A et/ou UV-B, différents de ceux de formule (1) et (2), sont choisis parmi les suivants : Ethylhexyl Salicylate, Ethylhexyl Methoxycinnamate, Octocrylene, Phenylbenzimidazole Sulfonic Acid, Terephthalylidene Dicamphor Sulfonic Acid, Benzophenone-3, Benzophenone-4, Benzophenone-5, 4-Methylbenzylidene Camphor, Benzimidazilate, Aniso-triazine, Ethylhexyl Triazone, Diethylhexyl Butamido Triazone, Methylene bis-Benzotriazolyl Tetramethylbutylphenol, Drometrizole Trisiloxane, et des mélanges de ceux-ci.

7. Composition cosmétique selon l'une quelconque des revendications précédentes, **caractérisée par le fait qu'**elle contient en outre un ou plusieurs pigments minéraux choisis parmi les nanopigments d'oxydes métalliques tels que l'oxyde de titane, l'oxyde de fer, l'oxyde de zinc, l'oxyde de zirconium et l'oxyde de cérium, éventuellement enrobés, et leurs mélanges.

8. Composition cosmétique selon l'une quelconque des revendications précédentes, **caractérisée par le fait qu'**elle contient en outre au moins un agent de bronzage et/ou de brunissage artificiel de la peau.

9. Composition cosmétique selon l'une quelconque des revendications précédentes, **caractérisée par le fait qu'**elle contient en outre un ou plusieurs adjuvants choisis parmi les corps gras, les solvants organiques, les agents épaississants, les agents antioxydants, les agents opacifiants, les agents stabilisants, les agents anti-mousse, les parfums, les agents conservateurs, les charges, les agents séquestrants, les agents propulseurs, les agents d'ajustement du pH, les colorants et les principes actifs cosmétiques.

10. Composition cosmétique pour la protection de la peau contre les rayons UV selon l'une quelconque des revendications précédentes, **caractérisée par le fait qu'**elle se présente sous forme de lotion, suspension, dispersion, émulsion, gel, gel-crème, pommade, bâtonnet solide, mousse aérosol ou spray.

11. Composition cosmétique pour la protection des cheveux contre les rayons UV selon l'une quelconque des revendications 1 à 9, **caractérisée par le fait qu'**elle se présente sous forme de shampooing, de lotion, de gel, de composition à rincer, de lotion ou gel coiffant ou traitant, de lotion ou gel pour le brushing ou la mise en plis, de composition de permanente ou de défrisage, de coloration ou de décoloration des cheveux ou de laque pour cheveux.

12. Composition cosmétique pour le maquillage des cils, des sourcils, de la peau ou des cheveux selon l'une quelconque des revendications 1 à 9, **caractérisée par le fait qu'**elle se présente sous forme de crème de traitement de l'épiderme, fond de teint, bâton de rouge à lèvres, fard à paupières, fard à joues, mascara, ligneur ou gel colorant.

13. Composé de formule (2) 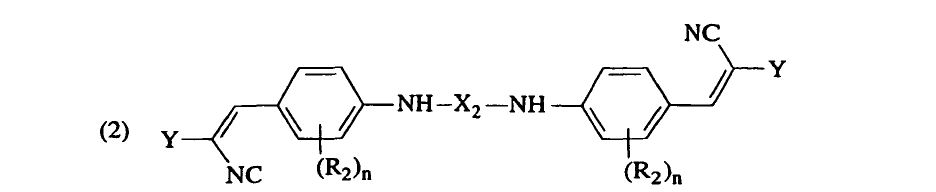 dans laquelle
X₂ représente un radical divalent de formule -(C=O)-R'₃-(C=O)-, -SO₂-R'₃-SO₂- ou -(C=O)-O-R'₃-O-(C=O)-,
Y représente un radical -(C=O)-R₄ ou -SO₂R₅,
R₂ représente un groupe alkyle en C₁₋₈, linéaire ou ramifié,
n vaut 0, 1 ou 2,
R'₃ représente une simple liaison ou un radical divalent alkylène en C₁₋₃₀ ou alcénylène en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,.
R₄ représente un radical -OR₆ ou -NHR₆,
R₅ représente un radical alkyle en C₁₋₃₀, linéaire ou ramifié, ou un noyau phényle non substitué ou substitué par des radicaux alkyle ou alcoxy en C₁₋₄,
R₆ représente un radical alkyle en C₁₋₃₀ ou alcényle en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium.

14. Procédé non-thérapeutique de protection de la peau et/ou des cheveux contre le rayonnement solaire, **caractérisé par le fait qu'**il consiste à appliquer sur la peau et/ou les cheveux une quantité efficace d'une composition cosmétique selon l'une quelconque des revendications 1 à 11.

## Patentansprüche

1. Kosmetische Zusammensetzung zur lokalen Anwendung, insbesondere zum Schutz der Haut und/oder des Haares vor Licht, wobei die Zusammensetzung in einem kosmetisch verträglichen Trägerstoff mindestens eine Verbindung der Formel (1) oder (2) enthält: 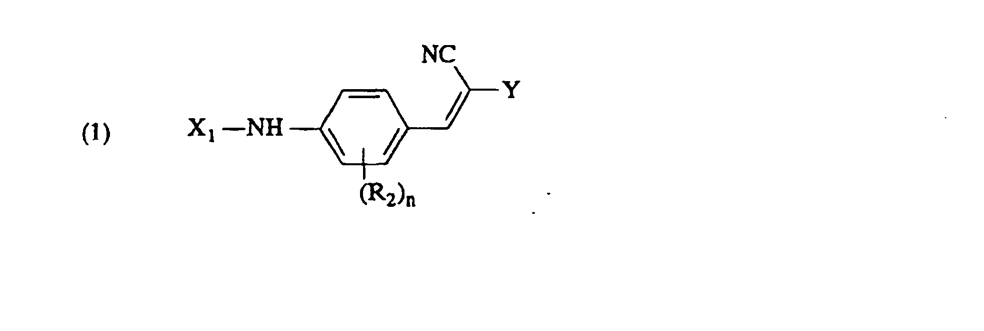 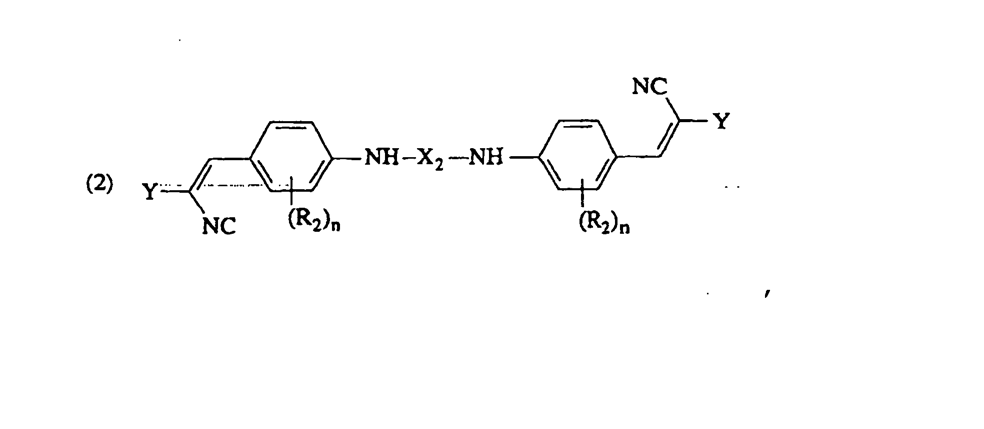 wobei
X₁ ein Radikal R₃- (C=O) -, R₃-SO₂- oder R₃-O- (C=O) - repräsentiert,
X₂ ein zweiwertiges Radikal der Formel -(C=O)-R'₃-(C=O) -, -SO₂-R' ₃-SO₂- oder - (C=O) -O-R' ₃-O- (C=O) - repräsentiert,
Y ein Radikal -(C=O)-R₄ oder -SO₂R₅ repräsentiert,
R₂ eine lineare oder verzweigte Alkylgruppe des Typs C₁₋₈ repräsentiert,
n gleich 0, 1 oder 2 ist,
R₃, linear oder verzweigt sein kann, ein Alkylradikal mit C₁₋₃₀) oder Alkenylradikal mit C₃₋₃₀ repräsentiert, das einen oder mehrere Hydroxylsubstituenten enthalten kann und das in der Kohlenstoffkette ein oder mehrere Heteroatome aufweisen kann, die aus der Gruppe Sauerstoff-, Stickstoff- und Siliziumatome ausgewählt sind,
R'₃ eine einfache Verbindung oder ein bivalentes Radikal eines Alkylens mit C₁₋₃₀ oder eines Alkenyls mit C₃₋₃₀), linear oder verzweigt, repräsentiert, das eine oder mehrere Hydroxylsubstituenten enthalten kann und das in der Kohlenstoffkette ein oder mehrere Heteroatome aufweisen kann, die aus der Gruppe Sauerstoff-, Stickstoff- und Siliziumatome ausgewählt sind,
R₄ ein -OR₆Radikal oder -NHR₆ Radikal repräsentiert,
R₅ ein lineares oder verzweigtes Alkylradikal mit C₁₋₃₀ oder einen Phenylkern repräsentiert, der durch Alkyl- oder Alcoxylradikale mit C₁₋₄ nicht substituiert oder substituiert ist,
R₆ ein Alkylradikal mit C₁₋₃₀ oder Alkenylradikal mit C₃₋₃₀, seien diese linear oder verzweigt, repräsentiert, das eine oder mehrere Hydroxylsubstituenten tragen kann und das in der Kohlenstoffkette ein oder mehrere Heteroatome aufweisen kann, die aus den Atomen von Sauerstoff, Stickstoff und Silizium ausgewählt sind.

2. Kosmetische Zusammensetzung nach Anspruch 1, **dadurch gekennzeichnet, dass**
X₁ ein Radikal R₃- (C=O) - oder R₃-O- (C=O) - repräsentiert,
X₂ ein Radikal - (C=O) -CH₂-CH₂- (C=O) - repräsentiert,
Y ein Radikal -(C=O)-R₄ repräsentiert,
n gleich 0 ist,
R₃ ein lineares oder verzweigtes Alkylradikal in C₁₋₁₀ repräsentiert,
R₄ ein Radikal -O-R₆ repräsentiert und
R₆ ein lineares oder verzweigtes Alkylradikal in C₁₋₃₀ repräsentiert, das möglicherweise ein oder mehrere Atome von Silizium enthält.

3. Kosmetische Zusammensetzung nach Anspruch 1 oder 2, **dadurch gekennzeichnet, dass** die Verbindungen der Formel (1) oder (2) in einer Konzentration von 0,1 bis 20 Gew.-%, und vorzugsweise in einer Konzentration von 0,5 bis 10 Gew.-% bezüglich dem Gesamtgewicht der kosmetischen Zusammensetzung vorhanden sind.

4. Kosmetische Zusammensetzung nach einem der vorhergehenden Ansprüche, **dadurch gekennzeichnet, dass** diese außerdem einen oder mehrerer in dem UV-A- und/oder UV-B-Bereich wirksame organische Solarfilter enthält, die sich von jenen der Formel (1) oder (2) unterscheiden.

5. Kosmetische Zusammensetzung nach Anspruch 4, **dadurch gekennzeichnet, dass** die in dem UV-A- und/oder UV-B-Bereich wirksamen organischen Solarfilter, die sich von jenen der Formel (1) und (2) unterscheiden, aus der Gruppe ausgewählt sind, zu der gehören: die Salizylderivate, die Benzylidenkampferderivate, die Triazinderivate, die Benzophenonderivate, die Zimtderivate, die Derivate von β,β'-Diphenylacrylat, die Phenylbenzimidazolderivate, die Antranilderivate, die Imidazolinderivate, die Methylen-bis-(Hydroxyphenyl-benzotriazol)-Derivate, die p-Aminobenzolsäurederivate, die Hydrocarbonpolymerfilter und die Silikonfilter.

6. Kosmetische Zusammensetzung nach Anspruch 5, **dadurch gekennzeichnet, dass** die in dem UV-A- und/oder UV-B-Bereich wirksamen organischen Solarfilter, die sich von jenen der Formel (1) und (2) unterscheiden, aus der folgenden Gruppe ausgewählt sind: Ethylhexylsalizylat, Ethylhexylmethoxycinnamat, Octocrylen, Phenylbenzimidazolsulfonsäure, Terephthalyliden-Dikampfersulfonsäure, Benzophenon-3, Benzophenon-4, Benzophenon-5, 4-Methylbenzylidenkampfer, Benzimidazilat, Anisotriazin, Ethylhexyltriazon, Diethylhexylbutamidotriazon, Methylen-bis-Benzotriazolyl-Tetramethylbutylphenol, Drometrizoltrisiloxan und Gemischen aus diesen.

7. Kosmetische Zusammensetzung nach einem der vorhergehenden Ansprüche, **dadurch gekennzeichnet, dass** diese außerdem ein oder mehrere anorganische Pigmente enthält, die aus den Nanopigmenten von Metalloxiden, beispielsweise des Titanoxids, des Eisenoxids, des Zinkoxids, des Zirconiumoxids und des Ceroxids, möglicherweise umhüllt, und von deren Mischungen, ausgewählt sind.

8. Kosmetische Zusammensetzung nach einem der vorhergehenden Ansprüche, **dadurch gekennzeichnet, dass** diese außerdem wenigstens einen Wirkstoff zum natürlichen und/oder künstlichen Bräunen der Haut enthält.

9. Kosmetische Zusammensetzung nach einem der vorhergehenden Ansprüche, **dadurch gekennzeichnet, dass** diese außerdem einen oder mehrere Adjuvante enthält, die aus der Gruppe ausgewählt sind, zu der gehören: Fette, organische Lösungsmittel, Verdickungsmittel, Antioxidationsmittel, Eintrübungsmittel, Stabilisatoren, Schaumbremsmittel, Parfums, Konservierungsstoffe, Balaststoffe, Sequestrierstoffe, Treibgasmittel, pH-Wert-Einstellstoffe, Farbstoffe und kosmetischen Wirkstoffe.

10. Kosmetische Zusammensetzung für den Schutz der Haut gegen die UV-Strahlen nach einem der vorhergehenden Ansprüche, **dadurch gekennzeichnet, dass** diese in Form einer Lotion, Suspension, Dispersion, Emulsion, eines Gels, einer Gel-Creme, Salbe, festen Stäbchen, Sprühschaum oder Spray zubereitet ist.

11. Kosmetische Zusammensetzung für den Schutz des Haares gegen die UV-Strahlen nach einem der Ansprüche 1 bis 9, **dadurch gekennzeichnet, dass** diese in Form von Schampon, Lotion, Gel, Spülungsmischung, Lotion oder Gel zum Frisieren oder zur Pflege, Lotion oder Gel zum Fönen oder Wasserwellenlegen, Zusammensetzung zum Dauerwellen oder Entkrausen, Einfärben oder Entfärben des Haares, oder als Haarlack zubereitet ist.

12. Kosmetische Zusammensetzung für das Schminken der Wimpern, der Augenbrauen, der Haut oder des Haares nach einem der Ansprüche 1 bis 9, **dadurch gekennzeichnet, dass** diese in Form von Epidermispflegecreme, Make-up, Lippenstift, Lidschminke, Wangenschminke, Wimperntusche, Liner oder Gel-Farbstoff zubereitet ist.

13. Verbindung der Formel (2) 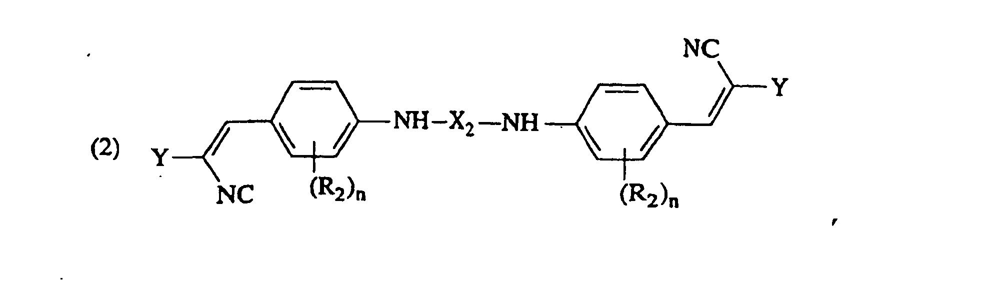 wobei
X₂ ein zweiwertiges Radikal der Formel -(C=O)-R'₃-(C=O) -, -SO₂-R'₃-SO₂- oder - (C=O) -O-R' ₃-O- (C=O) - repräsentiert,
Y ein Radikal -(C=O)-R₄ oder -SO₂R₅ repräsentiert,
R₂ eine lineare oder verzweigte Alkylgruppe des Typs C₁₋₈ repräsentiert,
n gleich 0, 1 oder 2 ist,
R'₃ eine einfache Verbindung oder ein bivalentes Alkandiylradikal in C₁₋₃₀ oder Alkenylen in C₃₋₃₀, seien diese linear oder verzweigt, repräsentiert, das einen oder mehrere Hydroxylsubstituenten tragen kann und das in der Kohlenstoffkette ein oder mehrere Heteroatome aufweisen kann, die unter den Atomen von Sauerstoff, Stickstoff und Silizium ausgewählt sind.
R₄ ein Radikal -OR₆ oder -NHR₆ repräsentiert,
R₅ ein lineares oder verzweigtes Alkylradikal in C₁₋₃₀ oder einen Phenylkern repräsentiert, der durch Alkyl- oder Alcoxylradikale in C₁₋₄ nicht substituiert oder substituiert sein kann,
R₆ ein Alkylradikal in C₁₋₃₀ oder Alkenylradikal in C₃₋₃₀ , seien diese linear oder verzweigt, repräsentiert, das einen oder mehrere Hydroxylsubstituenten tragen kann und in der Kohlenstoffkette ein oder mehrere Heteroatome aufweisen kann, die unter den Atomen von Sauerstoff, Stickstoff und Silizium ausgewählt sind.

14. Nicht therapeutisches Verfahren zum Schutz der Haut und/oder des Haares gegen Sonnenbestrahlung, **dadurch gekennzeichnet, dass** zu dem Verfahren der Schritt gehört: Auftragen einer wirksamen Menge einer kosmetischen Zusammensetzung nach einem der Ansprüche 1 bis 11 auf die Haut und/oder auf das Haar.

## Claims

1. Cosmetic composition for topical use, in particular intended for photoprotecting the skin and/or the hair, containing, in a cosmetically acceptable support, at least one compound of formula (1) or (2) 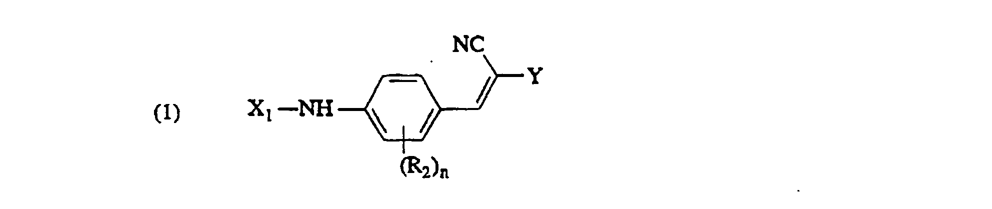 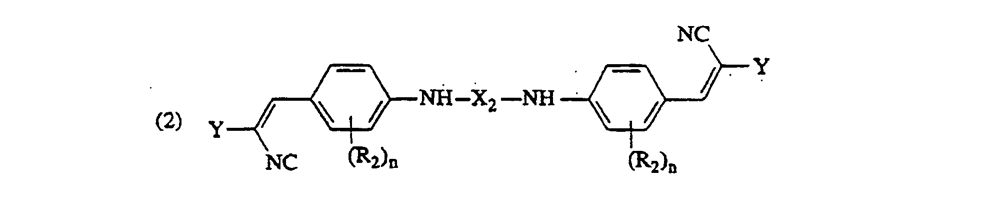 in which
X₁ represents a radical R₃- (C=O) -, R₃-SO₂- or R₃-O- (C=O) -,
X₂ represents a divalent radical of formula - (C=O) -R' ₃- (C=O) -, -SO₂-R'₃-SO₂- or - (C=O) -O-R'₃-O- (C=O) -,
Y represents a radical - (C=O) -R₄ or -SO₂R₅,
R₂ represents a linear or branched C₁₋₈ alkyl group,
n is 0, 1 or 2,
R₃ represents a linear or branched C₁₋₃₀ alkyl or C₃₋₃₀ alkenyl radical, possibly bearing one or more hydroxyl substituents and possibly containing, in the carbon chain, one or more hetero atoms chosen from oxygen, nitrogen and silicon atoms,
R'₃ represents a single bond or a linear or branched divalent C₁₋₃₀ alkylene or C₃₋₃₀ alkenylene radical, possibly bearing one or more hydroxyl substituents and possibly containing, in the carbon chain, one or more hetero atoms chosen from oxygen, nitrogen and silicon atoms,
R₄ represents a radical -OR₆ or -NHR₆,
R₅ represents a linear or branched C₁₋₃₀ alkyl radical or a phenyl nucleus that is unsubstituted or substituted with C₁₋₄ alkyl or alkoxy radicals,
R₆ represents a linear or branched C₁₋₃₀ alkyl or C₃₋₃₀ alkenyl radical, possibly bearing one or more hydroxyl substituents and possibly containing, in the carbon chain, one or more hetero atoms chosen from oxygen, nitrogen and silicon atoms.

2. Cosmetic composition according to Claim 1, **characterized in that**
X₁ represents a radical R₃- (C=O) - or R₃-O- (C=O) -,
X₂ represents a radical -(C=O)-CH₂-CH₂-(C=O)-,
Y represents a radical -(C=O)-R₄,
n is 0,
R₃ represents a linear or branched C₁₋₁₀ alkyl radical,
R₄ represents a radical -O-R₆, and
R₆ represents a linear or branched C₁₋₃₀ alkyl radical optionally containing one or more silicon atoms.

3. Cosmetic composition according to Claim 1 or 2, **characterized in that** the compound(s) of formula (1) or (2) is(are) present in a proportion of from 0.1% to 20% by weight and preferably in a proportion of from 0.5% to 10% by weight relative to the total weight of the cosmetic composition.

4. Cosmetic composition according to any one of the preceding claims, **characterized in that** it also contains one or more organic sunscreens that are active in the UV-A and/or UV-B range, other than those of formula (1) or (2).

5. Cosmetic composition according to Claim 4, **characterized in that** the organic sunscreens that are active in the UV-A and/or UV-B range, other than those of formulae (1) and (2), are chosen from salicylic derivatives, benzylidenecamphor derivatives, triazine derivatives, benzophenone derivatives, cinnamic derivatives, β,β'-diphenylacrylate derivatives, phenylbenzimidazole derivatives, anthranilic derivatives, .imidazoline derivatives, methylenebis(hydroxyphenylbenzotriazole) derivatives, p-aminobenzoic acid derivatives, screening hydrocarbon polymers and screening silicones.

6. Cosmetic composition according to Claim 5, **characterized in that** the organic sunscreens that are active in the UV-A and/or UV-B range, other than those of formulae (1) and (2), are chosen from the following: ethylhexyl salicylate, ethylhexyl methoxycinnamate, octocrylene, phenylbenzimidazolesulphonic acid, terephthalylidenedicamphorsulphonic acid, benzophenone-3, benzophenone-4, benzophenone-5, 4-methylbenzylidenecamphor, benzimidazilate, anisotriazine, ethylhexyltriazone, diethylhexylbutamidotriazone, methylenebis(benzotriazolyl)tetramethylbutylphenol, drometrizoletrisiloxane, and mixtures thereof.

7. Cosmetic composition according to any one of the preceding claims, **characterized in that** it also contains one or more mineral pigments chosen from nanopigments of metal oxides such as titanium oxide, iron oxide, zinc oxide, zirconium oxide and cerium oxide, that are optionally coated, and mixtures thereof.

8. Cosmetic composition according to any one of the preceding claims, **characterized in that** it also contains at least one agent for artificially tanning and/or browning the skin.

9. Cosmetic composition according to any one of the preceding claims, **characterized in that** it also contains one or more adjuvants chosen from fatty substances, organic solvents, thickeners, antioxidants, opacifiers, stabilizers, antifoams, fragrances, preserving agents, fillers, sequestering agents, propellants, pH regulators, colorants, and cosmetic active principles.

10. Cosmetic composition for protecting the skin against UV rays, according to any one of the preceding claims, **characterized in that** it is in the form of a lotion, a suspension, a dispersion, an emulsion, a gel, a cream-gel, an ointment, a solid tube, an aerosol mousse or a spray.

11. Cosmetic composition for protecting the hair against UV rays, according to any one of Claims 1 to 9, **characterized in that** it is in the form of a shampoo, a lotion, a gel, a rinse-out composition, a styling or treating lotion or gel, a blow-drying or hairsetting lotion or gel, a permanent-waving, straightening, dyeing or bleaching composition for the hair or a hair lacquer.

12. Cosmetic composition for making up the eyelashes, the eyebrows, the skin or the hair according to any one of Claims 1 to 9, **characterized in that** it is in the form of an epidermal treatment cream, a foundation, a tube of lipstick, an eyeshadow, a face powder, a mascara, an eyeliner or a colouring gel.

13. Compound of formula (2) 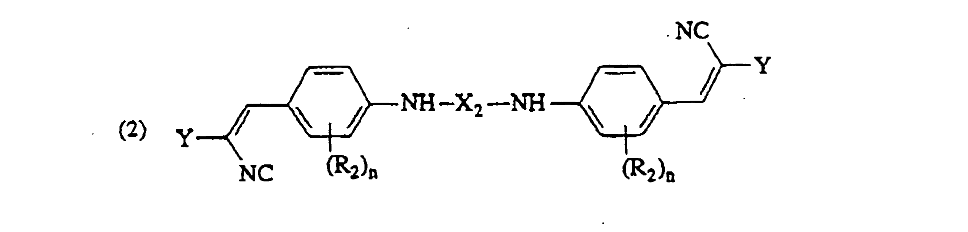 in which
X₂ represents a divalent radical of formula - (C=O) -R'₃- (C=O) -, -SO₂-R'₃-SO₂- or - (C=O) - O-R'₃-O- (C=O) -,
Y represents a radical - (C=O) -R₄ or -SO₂R₅,
R₂ represents a linear or branched C₁₋₈ alkyl group,
n is 0, 1 or 2,
R'₃ represents a single bond or a linear or branched divalent C₁₋₃₀ alkylene or C₃₋₃₀ alkenylene radical, possibly bearing one or more hydroxyl substituents and possibly containing, in the carbon chain, one or more hetero atoms chosen from oxygen, nitrogen and silicon atoms,
R₄ represents a radical -OR₆ or -NHR₆,
R₅ represents a linear or branched C₁₋₃₀ alkyl radical or a phenyl nucleus that is unsubstituted or substituted with C₁₋₄ alkyl or alkoxy radicals,
R₆ represents a linear or branched C₁₋₃₀ alkyl or C₃₋₃₀ alkenyl radical, possibly bearing one or more hydroxyl substituents and possibly containing, in the carbon chain, one or more hetero atoms chosen from oxygen, nitrogen and silicon atoms.

14. Non-therapeutic process for protecting the skin and/or the hair against solar radiation, **characterized in that** it consists in applying to the skin and/or the hair an effective amount of a cosmetic composition according to any one of Claims 1 to 11.
